# EUROPEAN PATENT SPECIFICATION

(11) **EP 4 343 354 B1**
(45) Date of publication and mention of the grant of the patent: **06.05.2026**
(21) Application number: 22913709.6
(22) Date of filing: 21.10.2022
(51) Int. Cl.: G01R 33/36, A61B 5/055

(54) **MAGNETIC RESONANCE SYSTEM AND CIRCUIT**
MAGNETRESONANZSYSTEM UND SCHALTUNG
SYSTÈME ET CIRCUIT À RÉSONANCE MAGNÉTIQUE

(30) Priority: 30.12.2021 CN 202111667375
(43) Date of publication of application: 27.03.2024
(73) Proprietor: Wuhan United Imaging Life Science Instrument Co., Ltd, Wuhan, Hubei 430206 (CN)
(72) Inventor: ZHANG, Yuman, Shanghai 201807 (CN); XU, Youlei, Shanghai 201807 (CN); ZHOU, Jianfan, Shanghai 201807 (CN); ZHAI, Renkuan, Wuhan, Hubei 430206 (CN)
(74) Representative: Wang, Bo
(86) International application number: PCT/CN2022/126764
(87) International publication number: WO 2023/124446

(56) References cited:
- EP-A2- 1 004 886
- WO-A1-2018/080291
- WO-A1-2021/112454
- CN-A- 104 769 452
- CN-A- 112 327 232
- CN-A- 113 608 155
- CN-A- 114 325 522
- JP-A- H03 165 741
- JP-A- H10 277 009
- US-A1- 2008 231 274
- ABOU-KHOUSA MOHAMED A ET AL: "Wideband RF Transmit-Receive Switch for Multi-Nuclei NMR Spectrometers", IEEE TRANSACTIONS ON CIRCUITS AND SYSTEMS II: EXPRESS BRIEFS, IEEE, USA, vol. 69, no. 3, 19 October 2021 (2021-10-19), pages 904 - 908, XP011902859, ISSN: 1549-7747, [retrieved on 20220315], DOI: 10.1109/TCSII.2021.3121210
- CHOI CHANG-HOON ET AL: "Design and construction of a novel1H/19F double-tuned coil system using PIN-diode switches at 9.4T", JOURNAL OF MAGNETIC RESONANCE, ACADEMIC PRESS, ORLANDO, FL, US, vol. 279, 7 April 2017 (2017-04-07), pages 11 - 15, XP085023265, ISSN: 1090-7807, DOI: 10.1016/J.JMR.2017.04.005
- SALEH GAMEEL ET AL: "Dual Tuned Switch for Dual Resonance 1H/13C MRI Coil", 2021 IEEE INTERNATIONAL IOT, ELECTRONICS AND MECHATRONICS CONFERENCE (IEMTRONICS), IEEE, 21 April 2021 (2021-04-21), pages 1 - 7, XP033916842, DOI: 10.1109/IEMTRONICS52119.2021.9422627

## Description

### CROSS-REFERENCE RELATED TO APPLICATIONS

This application claims priority to Chinese application No. 202111667375.X filed on December 30, 2021.

### TECHNICAL FIELD

The present disclosure relates to the field of magnetic resonance, and in particular to magnetic resonance systems and magnetic resonance circuits.

### BACKGROUND

With the enhancement of magnetic resonance field strength, the optimization of coil, and the emergence of ultra-high-speed sequences, the research on magnetic resonance of other nuclides (e.g., hydrogen (1H), carbon (13C), nitrogen (15N), fluorine (19F), sodium (23Na), phosphorus (31P), xenon (129Xe), etc.) has also developed rapidly. For the detection of these nuclides, more comprehensive imaging and evaluation of tissues and organs (e.g., the brain, the heart, the lungs, and other tissues and organs) can be performed. To realize the transmission and reception of radio frequencies of different nuclides, the circuits for transmitting and receiving different nuclides in the magnetic resonance radio frequency power transceiver are not the same. However, the method for switching the circuits through a hardware switch to respectively realize functions of the transmission and reception increases the hardware cost of the magnetic resonance radio frequency transceiver, and the efficiency of the transmission and reception of the radio frequency is relatively low.

Accordingly, it is desirable to provide magnetic resonance systems and magnetic resonance circuits that can effectively reduce the hardware cost and improve the efficiency of the transmission and reception of the radio frequency.
ABOU-KHOUSA MOHAMED A ET AL: "Wideband RF Transmit-Receive Switch for MultiNuclei NMR Spectrometers", IEEE TRANSACTIONS ON CIRCUITS AND SYSTEMS II: EXPRESS BRIEFS, IEEE, USA, vol. 69, no. 3, 19 October 2021 (2021-10-19), pages 904-908, XP011902859, ISSN: 1549-7747, DOI: 10.1109/TCSll.2021.3121210, relates to a new design of wideband high-power TR switch which could be devised for multi-nuclei NMR spectrometers.
CHOI CHANG-HOON ET AL: "Design and construction of a novel1 H/19F double-tuned coil system using PIN-diode switches at 9.4T", JOURNAL OF MAGNETIC RESONANCE, ACADEMIC PRESS, ORLANDO, FL, US, vol. 279, 7 April 2017 (2017-04-07), pages 11-15, XP085023265, ISSN: 1090-7807, DOI: 10.1016/J.JMR.2017.04.005, relates to a novel double-tuned 1H/19F RF coil.
CN 113 608 155 A relates to a magnetic resonance multi-core radio frequency coil device, a control method, and a magnetic resonance imaging system.
SALEH GAMEEL ET AL: "Dual Tuned Switch for Dual Resonance 1 H/13C MRI Coil", 2021 IEEE INTERNATIONAL IOT, ELECTRONICS AND MECHATRONICS CONFERENCE (IEMTRONICS), IEEE, 21 April 2021 (2021-04-21), pages 1-7, XP033916842, DOI: 10.1109/IEMTRONICS52119.2021.9422627, relates to two transmit/receive switch designs for 7 Tesla magnetic resonance spectroscopic imaging.
EP 1 004 886 A2 relates to a magnetic imaging apparatus that includes an RF coil in electrical communication with an RF signal generator and a receiver through an interface circuit (E).

### SUMMARY

The invention is set out in the appended set of claims.

### BRIEF DESCRIPTION OF THE DRAWINGS

The present disclosure is further described in terms of exemplary embodiments. These exemplary embodiments are described in detail with reference to the drawings. These embodiments are non-limiting exemplary embodiments, in which like reference numerals represent similar structures throughout the several views of the drawings, and wherein:
FIG. 1 is a schematic diagram illustrating a structure of a magnetic resonance system according to some embodiments of the present disclosure;
FIG. 2 is a schematic diagram illustrating a structure of a magnetic resonance circuit according to some embodiments of the present disclosure;
FIG. 3 is a schematic diagram illustrating a structure of a magnetic resonance circuit according to some embodiments of the present disclosure;
FIG. 4 is a schematic diagram illustrating a structure of a magnetic resonance circuit according to some embodiments of the present disclosure;
FIG. 5 is a schematic diagram illustrating a structure of a magnetic resonance circuit according to some embodiments of the present disclosure;
FIG. 6 is a schematic diagram illustrating a structure of a magnetic resonance circuit according to some embodiments of the present disclosure;
FIG. 7 is a schematic diagram illustrating a structure of a magnetic resonance circuit according to some embodiments of the present disclosure;
FIG. 8 is a schematic diagram illustrating a structure of a magnetic resonance circuit according to some embodiments of the present disclosure;
FIG. 9 is a schematic diagram illustrating a structure of a magnetic resonance system according to some embodiments of the present disclosure; and
FIG. 10 is a schematic diagram illustrating a structure of a magnetic resonance system according to some embodiments of the present disclosure.

### DETAILED DESCRIPTION

In order to illustrate the technical solutions related to the embodiments of the present disclosure, a brief introduction of the drawings referred to in the description of the embodiments is provided below. Obviously, the drawings described below are only some examples or embodiments of the present disclosure. Those skilled in the art, without further creative efforts, may apply the present disclosure to other similar scenarios according to these drawings. Unless apparent from the locale or otherwise stated, like reference numerals represent similar structures or operations throughout the several views of the drawings.

It will be understood that the term "system," "device," "unit," and/or "module" used herein are one method to distinguish different components, elements, parts, sections, or assembly of different levels in ascending order. However, the terms may be displaced by another expression if they achieve the same purpose.

As used in the disclosure and the appended claims, the singular forms "a," "an," and/or "the" may include plural forms unless the content clearly indicates otherwise. In general, the terms "comprise," "comprises," and/or "comprising," "include," "includes," and/or "including," merely prompt to include steps and elements that have been clearly identified, and these steps and elements do not constitute an exclusive listing. The methods or devices may also include other steps or elements.

The flowcharts used in the present disclosure illustrate operations that systems implement according to some embodiments in the present disclosure. It is to be expressly understood, the operations of the flowchart may be implemented not in order. Conversely, the operations may be implemented in an inverted order, or simultaneously. Moreover, one or more other operations may be added to the flowcharts. One or more operations may be removed from the flowcharts.

The embodiments of the present disclosure provide a magnetic resonance system and a magnetic resonance circuit that can be applied to a magnetic resonance system supporting a plurality of nuclides, thereby realizing transmission and reception of the plurality of nuclides in a high field (e.g., magnetic field strength of 5T and above) magnetic resonance broadband. In some embodiments, the magnetic resonance system and the magnetic resonance circuit can be applied to a medical imaging system, a life science instrumentation, and any other system that uses magnetic resonance technology. In some embodiments, the medical imaging system may include a magnetic resonance imaging (MRI) system, a computed tomography-magnetic resonance imaging (MRI-CT) system, a positron emission tomography-magnetic resonance imaging (PET-MRI) system, a single photon emission computed tomography-magnetic resonance imaging (SPECT-MRI) system, a digital subtraction angiography-magnetic resonance imaging (DSA-MRI) system, etc.

In some embodiments, the magnetic resonance system includes a magnetic resonance transmitter, a magnetic resonance receiver, and a magnetic resonance radio frequency power transceiver. The magnetic resonance radio frequency power transceiver includes a communication circuit and a control circuit. The communication circuit provides a first communication circuit and a second communication circuit. The first communication circuit is configured to transmit one or more radio frequency signals to one or more coils. The second communication circuit is configured to transmit one or more magnetic resonance signals to the magnetic resonance receiver. The control circuit is configured to send one or more control signals to the communication circuit to control the first communication circuit or the second communication circuit to turn or cut off. The one or more radio frequency signals may be a plurality of radio frequency signals corresponding to different kinds of nuclides, and the one or more magnetic resonance signals may be a plurality of magnetic resonance signals transmitted by different kinds of nuclides. Compared to providing an independent transmission channel for the radio frequency signal or the magnetic resonance signal corresponding to each kind of nuclides, by adopting a magnetic resonance radio frequency power transceiver supporting broadband connectivity at multiple frequency points, a count of transmission channels between the magnetic resonance transmitter and the coil, as well as between the coil and the magnetic resonance receiver may be effectively reduced, thereby reducing the volume and the hardware cost of the magnetic resonance system, and improving operating efficiency of transmitting and receiving the radio frequency.

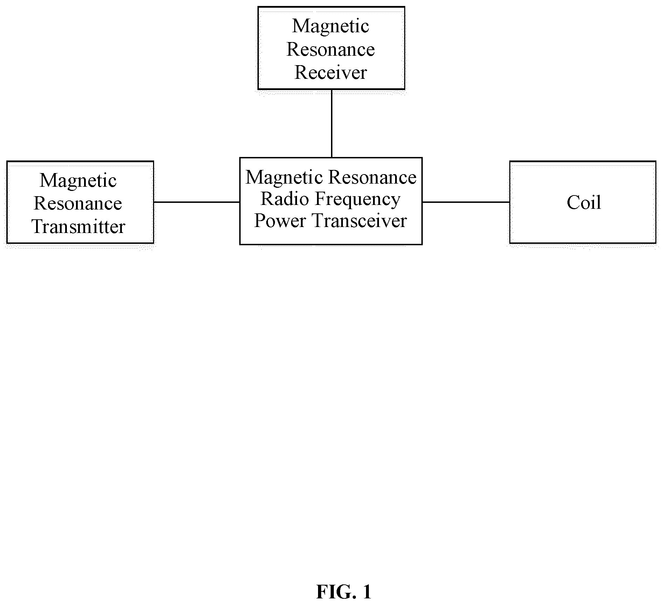

FIG. 1 is a schematic diagram illustrating a structure of a magnetic resonance system according to some embodiments of the present disclosure.

As shown in FIG. 1, the magnetic resonance system may include a magnetic resonance transmitter, a magnetic resonance receiver, a coil, and a magnetic resonance radio frequency power transceiver.

The magnetic resonance transmitter is connected to the magnetic resonance radio frequency power transceiver to generate one or more radio frequency signals at different moments or the same moment. The one or more radio frequency signals may be a plurality of radio frequency signals corresponding to different kinds of nuclides. In the magnetic resonance imaging technique, a radio frequency transmitting coil may be utilized to send a radio frequency signal to a detection object, thereby exciting a specific frequency of one or more nuclides in the detection object to resonate to generate one or more magnetic resonance signals. Since resonance frequencies of different kinds of nuclides are different, it is necessary to use different frequencies of radio frequency signals to excite different kinds of nuclides to generate different magnetic resonance signals. The detection of the relevant nuclides may be used to perform more comprehensive imaging and assessment on tissues and organs (e.g., the brain, the heart, the lungs, etc.). In some embodiments, different kinds of nuclides may include hydrogen (1H), carbon (13C), nitrogen (15N), fluorine (19F), sodium (23Na), phosphorus (31P), xenon (129Xe), etc.

The magnetic resonance radio frequency power transceiver is respectively connected to the magnetic resonance transmitter, the magnetic resonance receiver, and the coil to be configured to control the transmission and reception of the radio frequency of the magnetic resonance system. The magnetic resonance radio frequency power transceiver includes a transmitting state and a receiving state. When the magnetic resonance radio frequency power transceiver is in the transmitting state, a transmission channel between the magnetic resonance transmitter and the coil is turned on, and the transmission channel between the coil and the magnetic resonance receiver is cut off. When the magnetic resonance radio frequency power transceiver is in the receiving state, the transmission channel between the coil and the magnetic resonance receiver is turned on, and the transmission channel between the magnetic resonance transmitter and the coil is cut off.

The coil is connected to the magnetic resonance radio frequency power transceiver and configured to generate a magnetic field that excites resonance of different kinds of nuclides or collect one or more magnetic resonance signals, wherein the one or more magnetic resonance signals may be a plurality of magnetic resonance signals transmitted by different kinds of nuclides. In some embodiments, when the magnetic resonance radio frequency power transceiver is in the transmitting state, the transmission channel between the magnetic resonance transmitter and the coil is turned on, at which time the plurality of radio frequency signals corresponding to the different kinds of nuclides generated by the magnetic resonance transmitter is transmitted to the coil through the transmission channel, and the coil generates a magnetic field that excites resonance of the different kinds of nuclides, causing corresponding different kinds of nuclides to generate magnetic resonance signals under an action of the magnetic field. In some embodiments, the magnetic field that excites the resonance of the different kinds of nuclides may include a circularly polarized magnetic field generated by orthogonal excitation. In some embodiments, when the magnetic resonance radio frequency power transceiver is in the receiving state, the transmission channel between the coil and the magnetic resonance receiver is turned on, at which time the coil is used to collect the plurality of magnetic resonance signals emitted by the different kinds of nuclides. In some embodiments, the coil may be provided inside the magnetic resonance system or may be connected to the magnetic resonance radio frequency power transceiver inside the magnetic resonance system as an external component. In some embodiments, the coil may include multiple groups of coils, and each group of coils corresponds to the radio frequency signals or the magnetic resonance signals of the different kinds of nuclides, i.e., each group of coils is respectively used to emit (receive) the radio frequency signals (magnetic resonance signals) of a particular kind of nuclide.

The magnetic resonance receiver is connected to the magnetic resonance radio frequency power transceiver to receive one or more magnetic resonance signals collected by the coil at different moments or the same moment. In some embodiments, when the magnetic resonance radio frequency power transceiver is in the receiving state, the transmission channel between the coil and the magnetic resonance receiver is turned on, and the magnetic resonance signals collected by the coil are transmitted to the magnetic resonance receiver through the transmission channel, which may be imaged after subsequent processing.

In some embodiments, the magnetic resonance radio frequency power transceiver may switch the transmitting state or the receiving state based on a control signal generated by the magnetic resonance system. Merely by way of example, the magnetic resonance radio frequency power transceiver may be set to the transmitting state based on the control signal, i.e., to enter a first operating mode in which the magnetic resonance system may transmit the radio frequency signals generated by the magnetic resonance transmitter to the coil. The magnetic resonance radio frequency power transceiver may be set to the receiving state according to the control signal, i.e., to enter a second operating mode in which the magnetic resonance system may transmit the magnetic resonance signals collected by the coil to the magnetic resonance receiver.

In some embodiments, the magnetic resonance radio frequency power transceiver may provide the same transmission channel for the plurality of radio frequency signals corresponding to the different kinds of nuclides. For example, the plurality of radio frequency signals corresponding to the different kinds of nuclides generated by the magnetic resonance transmitter may be transmitted to the coil through the same transmission channel provided by the magnetic resonance radio frequency power transceiver. In this case, the transmission of the plurality of radio frequency signals corresponding to the different kinds of nuclides may be realized by merely setting a switch for the same transmission channel, without setting a switch for a specific frequency or broadband corresponding to each kind of nuclides, thereby effectively reducing redundant hardware switches. In some embodiments, the magnetic resonance transmitter may simultaneously generate radio frequency signals corresponding to the same kind of nuclide, or the magnetic resonance transmitter may generate a plurality of radio frequency signals corresponding to different kinds of nuclides at different times, and then the radio frequency signals may be transmitted by the same radio frequency power transceiver.

In some embodiments, the magnetic resonance radio frequency power transceiver may enter the first operating mode or the second operating mode according to a control signal, thereby realizing the switching between the transmission channel between the magnetic resonance transmitter and the coil and the transmission channel between the coil and the magnetic resonance receiver transmission channel, without providing different transmission channels for the excitation (or reception) of different nuclides, avoiding the use of excessive hardware switches, and solving a problem of excessive hardware switches, relatively high cost, and relatively low efficiency of the transmission and reception of the radio frequency of the magnetic resonance receiver in the related technology, reducing the volume and the hardware cost of the magnetic resonance device, and improving operating efficiency.

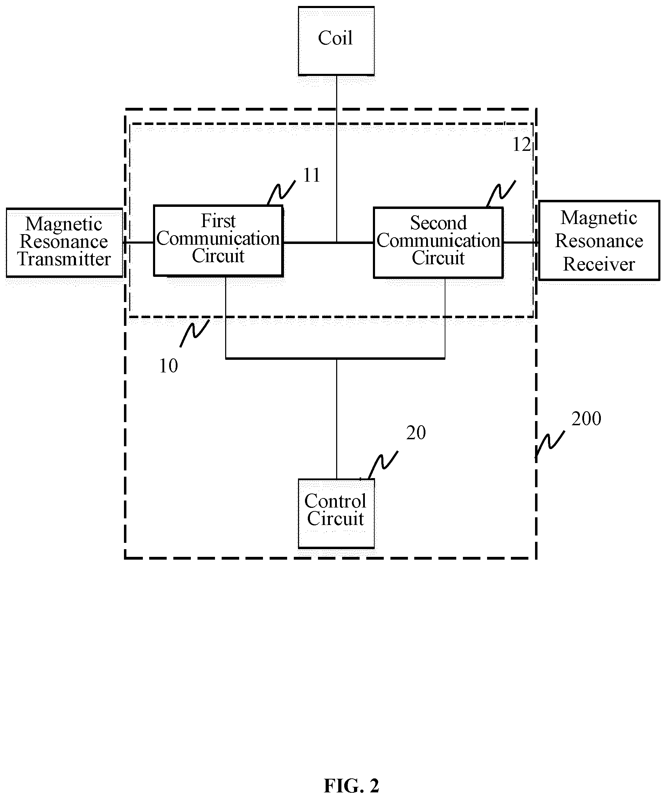

FIG. 2 is a schematic diagram illustrating a structure of a magnetic resonance circuit according to some embodiments of the present disclosure.

As shown in FIG. 2, a magnetic resonance circuit 200 is a magnetic resonance radio frequency power transceiver. The magnetic resonance circuit 200 is applied to a magnetic resonance system including a magnetic resonance transmitter, a magnetic resonance receiver, and a coil. The magnetic resonance circuit 200 includes a communication circuit 10 and a control circuit 20. The communication circuit 10 includes a first communication circuit 11 and a second communication circuit 12. The first communication circuit 11 is used to transmit one or more radio frequency signals to the coil, the second communication circuit 12 is used to transmit one or more magnetic resonance signals to the magnetic resonance receiver, and the control circuit 20 is used to send a control signal to control the first communication circuit 11 or the second communication circuit 12 to turn on or cut off. The control signal includes a turn-on signal and/or a cut-off signal. Specifically, the first communication circuit 11 may be used to transmit a plurality of radio frequency signals corresponding to different kinds of nuclides to the coil, and the second communication circuit 12 is used to transmit a plurality of magnetic resonance signals emit by the different kinds of nuclides to the magnetic resonance receiver. The control circuit 20 includes a power supply, and the power supply is used to output the turn-on signal and/or the cut-off signal.

As shown in FIG. 2, one end of the first communication circuit 11 is connected to the magnetic resonance transmitter, and another end of the first communication circuit 11 is connected to the second communication circuit 12. One end of the second communication circuit 12 is connected to the magnetic resonance receiver. The coil is connected to one end where the first communication circuit 11 and the second communication circuit 12 are connected, and the control circuit 20 is connected to the first communication circuit 11 and the second communication circuit 12, respectively.

In some embodiments, the control circuit 20 may receive a system instruction of the magnetic resonance system, generate a control signal based on the system instruction, and send the control signal to the communication circuit 10 to control the first communication circuit 11 in the communication circuit 10 to turn on or cut off, as well as the second communication circuit 12 to turn on or cut off. Specifically, the first communication circuit 11 is used to connect the magnetic resonance transmitter and the coil to send the radio frequency signals output from the magnetic resonance transmitter to the coil, and the second communication circuit 12 is used to connect the coil and the magnetic resonance receiver to send the magnetic resonance signals collected by the coil to the magnetic resonance receiver. In some embodiments, the system instruction may be automatically generated by the magnetic resonance system or manually input by a user, and the user may control the communication circuit 10 to turn on or cut off through the system instruction.

In some embodiments, the first communication circuit 11 and the second communication circuit 12 may be provided with a first switch and a second switch, respectively. The closure of the first switch or closure of the second switch is realized through the control signal sent by the control circuit 20, thereby realizing the turn-on of the first communication circuit 11 or the turn-on of the second communication circuit 12. In some embodiments, the communication circuit 10 may be provided with a dual-channel selection switch. The dual-channel selection switch is connected to the first communication circuit 11 and the second communication circuit 12, respectively. The closure of one channel of the dual-channel selection switch and the cut off of another channel of the dual-channel selection switch are realized by the control signal sent by the control circuit 20, thereby realizing the turn-on of the first communication circuit 11 or the turn-on of the second communication circuit 12.

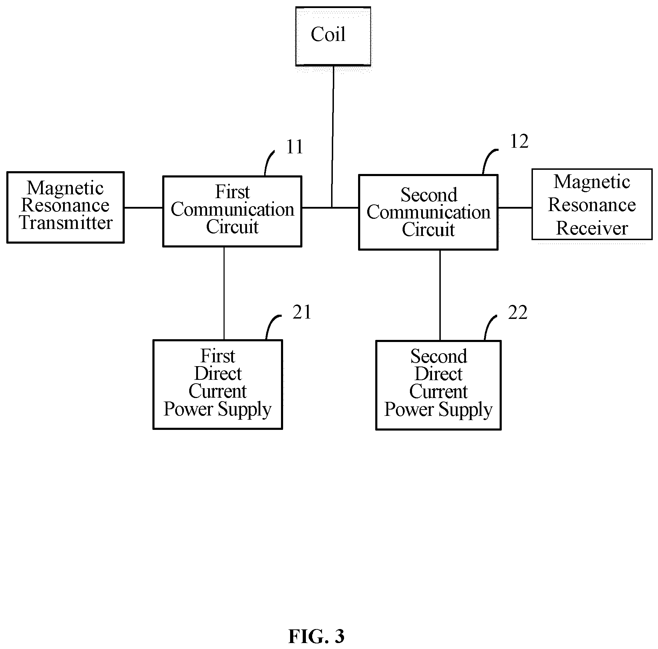

FIG. 3 is a schematic diagram illustrating a structure of a magnetic resonance circuit according to some embodiments of the present disclosure.

As shown in FIG. 3, the control circuit 20 includes a first direct current power supply 21 and a second direct current power supply 22. The first direct current power supply 21 is connected to the first communication circuit 11, and the second direct current power supply 22 is connected to the second communication circuit 12. The first direct current power supply 21 is used to output a first turn-on signal and/or a first cut-off signal, and the second direct current power supply 22 is used to output a second cut-off signal and/or a second turn-on signal to control the first communication circuit 11 or the second communication circuit 12 to turn on or cut off. In some embodiments, when the magnetic resonance radio frequency power transceiver is required to be set to a transmitting state, the first direct current power supply 21 may output the first turn-on signal and the second direct current power supply 22 may output the second cut-off signal, at this time, the control circuit 20 may control the first communication circuit 11 to turn on and control the second communication circuit 12 to cut off. In some embodiments, when the magnetic resonance radio frequency power transceiver is required to be set to a receiving state, the first direct current power supply 21 may output the first cut-off signal, and the second direct current power supply 22 may output the second direct communication signal, at this time, the control circuit 20 may control the first communication circuit 11 to cut off and control the second communication circuit 12 to turn on.

In some embodiments, the control signal output by the control circuit 20 may include a first control signal and a second control signal. The first control signal may be a superposition signal of the first turn-on signal and the second cut-off signal to cause the first communication circuit 11 to turn on and the second communication circuit 12 to cut off. At this time, the communication circuit 10 is used to transmit the radio frequency signals sent by the magnetic resonance transmitter to the coil. The second control signal may be a superposition signal of the first cut-off signal and the second turn-on signal to cause the first communication circuit 11 to turn off and the second communication circuit 12 to turn on. At this time, the communication circuit 10 is used to transmit the magnetic resonance signals collected by the coil to the magnetic resonance receiver.

In some embodiments, by controlling the turn on and cut off of the first communication circuit 11 and the second communication circuit 12 by the first direct current power supply 21 and the second direct current power supply 22, respectively, there is no need for a complicated circuit control structure, and the switching of the communication circuit 10 can be realized by only switching the turn-on signal and the cut-off signal output from the first direct current power supply 21 and the second direct current power supply 22, thereby further realizing the switching between the transmission channel between the magnetic resonance transmitter and the coil and the transmission channel between the coil and the magnetic resonance receiver, and the process of realizing the switching is simple and the hardware cost of the control circuit 20 is reduced.

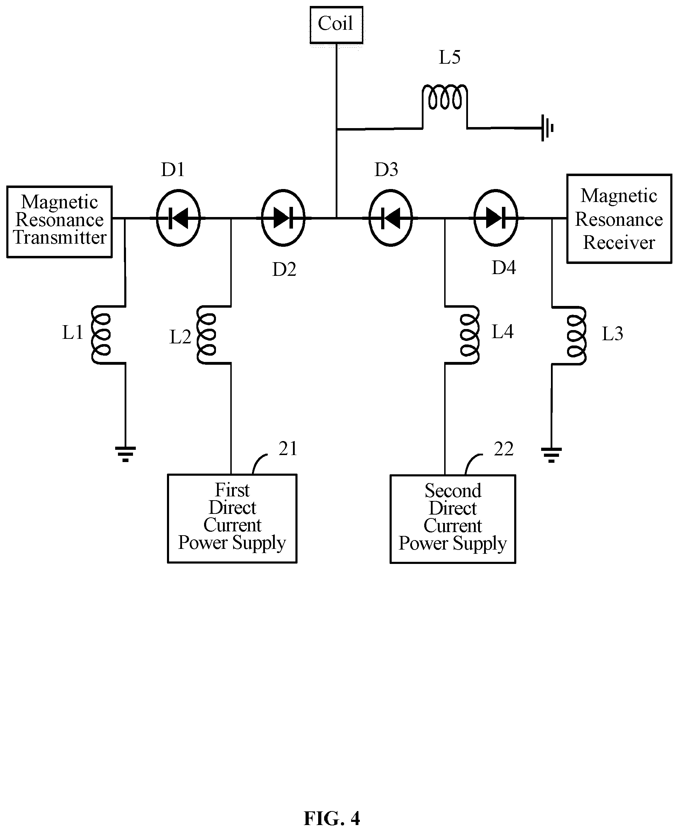

FIG. 4 is a schematic diagram illustrating a structure of a magnetic resonance circuit according to some embodiments of the present disclosure.

As shown in FIG. 4, the first communication circuit 11 includes a first diode D1 and a second diode D2, and the second communication circuit 12 includes a third diode D3 and a fourth diode D4. One end (the negative electrode) of the first diode D1 is connected to the magnetic resonance transmitter, one end (the positive electrode) of the second diode D2 is connected to another end (the positive electrode) of the first diode D1, and another end (the negative electrode) of the second diode D2 is connected to the coil. One end (the negative electrode) of the third diode D3 is connected to the coil, one end (the positive electrode) of the fourth diode D4 is connected to another end (the positive electrode) of the third diode D3, and another end (the negative electrode) of the fourth diode D4 is connected to the magnetic resonance receiver. The diode refers to a diode with a switching function, for example, a photodiode. In some embodiments, the diode may include one or more of a pass-switching diode, a high-speed switching diode, an ultra-high-speed switching diode, a low-power switching diode, a high-reverse-voltage switching diode, a silicon voltage switching diode, etc. In some embodiments, in order to meet power requirements of the communication circuit 10 for exciting different nuclides, one or more of the diodes (e.g., the first diode D1, the second diode D2, the third diode D3, and the fourth diode D4) need to be able to withstand a certain voltage and a certain current. Merely by way of example, the first diode D1 may withstand a maximum high voltage of 3000V and a current of 25A, and a minimum voltage of 230V and a current of 8A. Optionally, in order to be suitable for the magnetic resonance environment of magnetic resonance system applications, the diodes in the communication circuit 10 are non-magnetic structures.

Merely by way of example, the diode is a switching diode, and the first diode D1, the second diode D2, the third diode D3, and the fourth diode D4 are provided in the magnetic resonance circuit based on the characteristics of forward-biased conduction and reverse-biased cutoff of the switching diode. Specifically, the first diode D1 and the second diode D2 form the first communication circuit 11. When the first direct current power supply 21 inputs the first turn-on signal, the first diode D1 and the second diode D2 are in a turn-on state; and when the first direct current power supply 21 inputs the first cut-off signal, the first diode D1 and the second diode D2 are in a cut-off state. The third diode D3 and the fourth diode D4 form the second communication circuit 12. When the second direct current power supply 22 inputs the second turn-on signal, the third diode D3 and the fourth diode D4 are in the turn-on state; and when the second direct current power supply 22 inputs the second cut-off signal, the third diode D3 and the fourth diode D4 are in the cut-off state.

In some embodiments, the first turn-on signal and the second turn-on signal are positive voltages that exceed a turn-on voltage of the switching diode. The first cut-off signal and the second cut-off signal are negative voltages below a cut-off voltage of the diode. The numerical magnitudes of the first cut-off signal and the second cut-off signal may be set based on a radio frequency power amplifier in the magnetic resonance system. It should be appreciated that the first turn-on signal and the second turn-on signal in this embodiment may be set to exceed the turn-on voltage of the switching diode to drive the switching diode to turn on. The first cut-off signal and the second cut-off signal may be set to be lower than the cut-off voltage of the switching diode to cut off the switching diode. In some embodiments, the first turn-on signal is a positive voltage that exceeds the turn-on voltage of the first diode D1 and the turn-on voltage of the second diode D2, and the second turn-on signal is a positive voltage that exceeds the turn-on voltage of the third diode D3 and the turn-on voltage of the fourth diode D4. The first cut-off signal is a negative voltage below the cut-off voltage of the first diode D1 and the cut-off voltage of the second diode D2, and the second cut-off signal is a negative voltage below the cut-off voltage of the third diode D3 and the cut-off voltage of the fourth diode D4.

Merely by way of example, the first direct current power supply 21 inputs the first turn-on signal and the second direct current power supply 22 inputs the second cut-off signal, at which time the first diode D1 and the second diode D2 are in the turn-on state and the third diode D3 and the fourth diode D4 are in the cut-off state, i.e., the first communication circuit 11 is turned on, and the second communication circuit 12 is cut off. At this time, the magnetic resonance system can be used to transmit the radio frequency signals sent by the magnetic resonance transmitter to the coil to further generate the magnetic resonance signals. The first direct current power supply 21 inputs the first cut-off signal, and the second direct current power supply 22 inputs the second turn-on signal, at which time the first diode D1 and the second diode D2 are in the cut-off state, and the third diode D3 and the fourth diode D4 are in the turn-on state, i.e., the first communication circuit 11 is cut off, and the second communication circuit 12 is turned on. At this time, the magnetic resonance system can be used to transmit the magnetic resonance signals collected by the coil to the magnetic resonance receiver for subsequent processing.

In some embodiments, based on the property that an inductor passes direct current and blocks alternating current, the magnetic resonance circuit may further include an inductor element. The inductor element is connected among the magnetic resonance transmitter, the coil, the magnetic resonance receiver, the communication circuit, and the control circuit. In some embodiments, the inductor element includes a first inductor L1, a third inductor L3, and a fifth inductor L5. One end of the first inductor L1 is connected to the magnetic resonance transmitter, and another end of the first inductor L1 is grounded. One end of the third inductor L3 is connected to the magnetic resonance receiver, and another end of the third inductor L3 is grounded. One end of the fifth inductor L5 is connected to the coil, and another end of the fifth inductor L5 is grounded. In some embodiments, the inductor element further includes a second inductor L2 and a fourth inductor L4. The second inductor L2 is connected between the first direct current power supply 21 and the first communication circuit 11, and the fourth inductor L4 is connected between the second direct current power supply 22 and the second communication circuit 12. Specifically, one end of the second inductor L2 is connected to the first direct current power supply 21, and another end of the second inductor L2 is respectively connected to the positive electrode of the first diode D1 and the positive electrode of the second diode D2. One end of the fourth inductor L4 is connected to the second direct current power supply 22, and another end of the fourth inductor L4 is respectively connected to the positive electrode of the third diode D3 and the positive electrode of the fourth diode D4.

The first inductor L1 and the second inductor L2 may be used to isolate the radio frequency signals output by the magnetic resonance transmitter, and may also be used to conduct the direct current output by the first direct current power supply 21. The third inductor L3 and the fourth inductor L4 may be used to isolate collected signals output by the coil, and may also be used to conduct the direct current output by the second direct current power supply 22. The fifth inductor L5 may be used to isolate the radio frequency signals output by the magnetic resonance transmitter and the magnetic resonance signals output by the coil, and may also be used to conduct the direct current output by the first direct current power supply 21 and the direct current output by the second direct current power supply 22.

In some embodiments, when the first communication circuit 11 is conducted, since the first direct current power supply 21 outputs the direct current and the radio frequency signal output by the magnetic resonance transmitter is an alternating current signal, the first inductor L1, the second inductor L2, and the fifth inductor L5 conduct the direct current output by the first direct current power supply 21 and isolate the transmission signal sent by the magnetic resonance transmitter to the coil, thereby avoiding loss of the transmission signal during transmission. When the second communication circuit 12 is conducted, since the second direct current power supply 22 outputs the direct current and the magnetic resonance signal collected by the coil is the alternating current signal, the third inductor L3, the fourth inductor L4, and the fifth inductor L5 conduct the direct current output by the second direct current power supply 22 and isolate the collected signal sent by the coil to the magnetic resonance receiver, thereby avoiding the loss of the collected signal during transmission.

In the embodiments of the present disclosure, the communication circuit 10 is formed by the first diode D1, the second diode D2, the third diode D3, and the fourth diode D4, such that the switching between the conduction of the first communication circuit 11 and the conduction of the second communication circuit 12 is realized according to the turn-on signals or the cut-off signals of the direct current power supply 21 and the second direct current power supply 22. The above scheme is easy to implement without manual operation. Further, the first inductor L1, the second inductor L2, the third inductor L3, the fourth inductor L4, and the fifth inductor L5 are disposed in the magnetic resonance circuit to isolate the radio frequency signals and the collected magnetic resonance signals during transmission, which avoids the loss of the radio frequency signals and the collected magnetic resonance signals during transmission to ensure signal quality, thereby improving the detection accuracy of the magnetic resonance system.

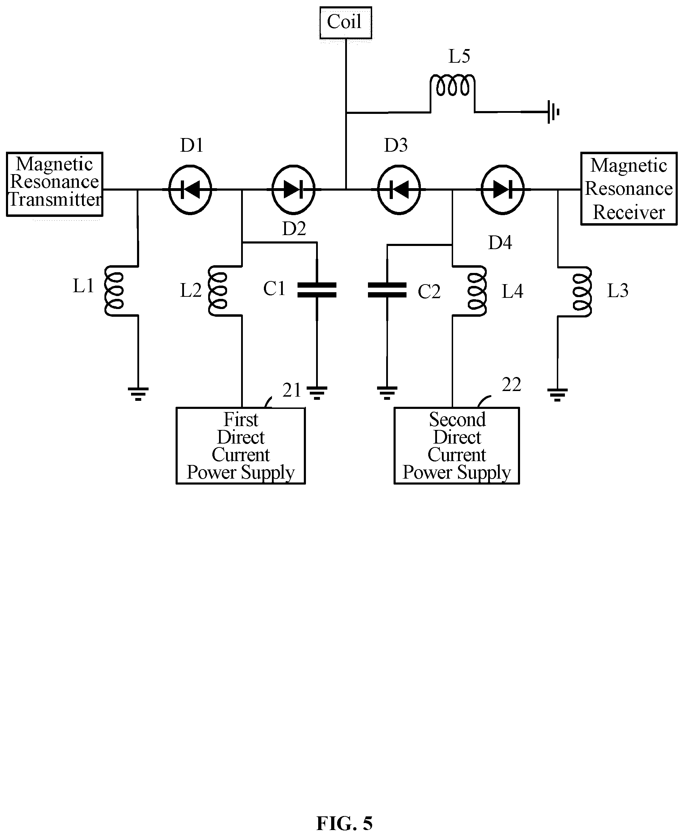

FIG. 5 is a schematic diagram illustrating a structure of a magnetic resonance circuit according to some embodiments of the present disclosure.

As shown in FIG. 5, the magnetic resonance circuit may further include a capacitive element. The capacitive element, together with the second inductor L2 and the fourth inductor L4, may form a filter circuit for filtering out interferences from signals other than the control signal, the plurality of radio frequency signals, and the plurality of magnetic resonance signals. In some embodiments, the capacitive element may include a first capacitor C1 and a second capacitor C2. One end of the first capacitor C1 is connected to the second inductor L2, and another end of the first capacitor C1 is grounded. One end of the second capacitor C2 is connected to the fourth inductor L4, and another end of the second capacitor C2 is grounded. The first capacitor C1 and the second inductor L2 form a first filter circuit. The second capacitor C2 and the fourth capacitor L4 form a second filter circuit. In some embodiments, to meet the requirements of the magnetic resonance circuit for filtering out interferences from other signals, and to realize the transmission and reception of multiple kinds of nucides under an ultra-high field magnetic resonance broadband, the capacitive element (e.g., the first capacitor C1 and the second capacitor C2) needs to have a high voltage resistance and a good radio frequency characteristic. In some embodiments, the capacitive element may be an RF high-power capacitor.

In some embodiments, the first filter circuit composed of the first capacitor C1 and the second inductor L2 is a low-pass filter circuit, and the first filter circuit may be used to filter out the interferences from other signals to ensure that the control signal of the first direct current power supply can be transmitted to the diode through the filter circuit, and at the same time to ensure that the radio frequency signals generated by the magnetic resonance transmitter can arrive at the coil completely.

In some embodiments, the second filter circuit formed by the second capacitor C2 and the fourth inductor L4 is also a low-pass filter circuit, and the second filter circuit may be used to filter out the interferences from other signals to ensure that the control signal of the second direct current power supply can be transmitted to the diode through the filter circuit, and at the same time to ensure that the magnetic resonance signal collected by the coil can arrive at the magnetic resonance receiver completely.

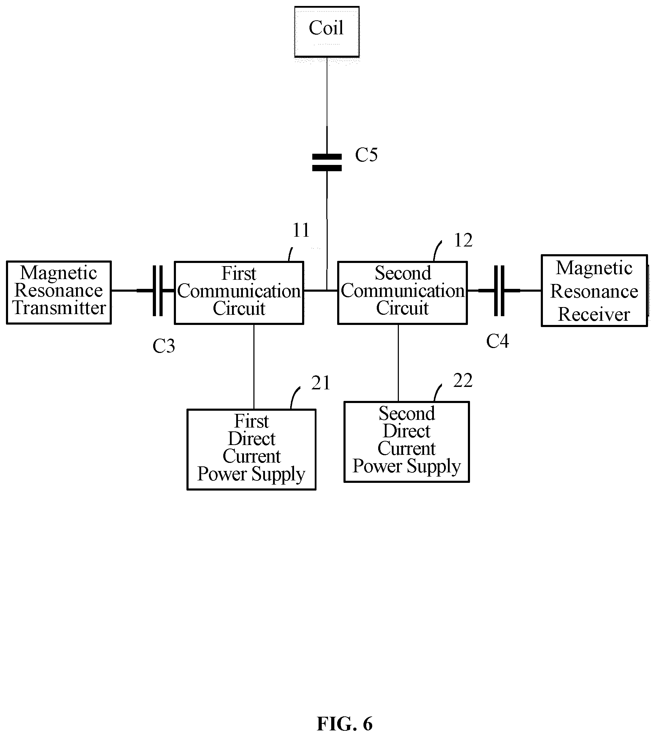

FIG. 6 is a schematic diagram illustrating a structure of a magnetic resonance circuit according to some embodiments of the present disclosure.

As shown in FIG. 6, in some embodiments, based on the property that a capacitor passes the alternating current and blocks the direct current, the magnetic resonance circuit may include a third capacitor C3, a fourth capacitor C4, and a fifth capacitor C5. One end of the third capacitor C3 is connected to the magnetic resonance transmitter, and another end of the third capacitor C3 is connected to the first communication circuit 11. One end of the fourth capacitor C4 is connected to the magnetic resonance receiver, and another end of the fourth capacitor C4 is connected to the second communication circuit 12. One end of the fifth capacitor C5 is connected to the coil, and another end of the fifth capacitor C5 is respectively connected to the first communication circuit 11 and the second communication circuit 12. The third capacitor C3 may be used to isolate the direct current output by the first direct current power supply 21, the fourth capacitor C4 may be used to isolate the direct current output by the second direct current power supply 22, and the fifth capacitor C5 may be used to isolate the direct current output by the first direct current power supply 21 and the direct current output by the second direct current power supply 22. In some embodiments, the third capacitor C3, the fourth capacitor C4, or the fifth capacitor C5 may also be an RF high-power capacitor.

In some embodiments, when the first communication circuit 11 is conducted, the third capacitor C3 may be used to isolate a current output by the first direct current power supply 21 to avoid the current passing through the magnetic resonance transmitter. When the second communication circuit 12 is conducted, the fourth capacitor C4 may be used to isolate the current output from the second direct current power supply 22 to avoid the current passing through the magnetic resonance receiver. The fifth capacitor C5 may be used to isolate the current output from the first direct current power supply 21 and the current output from the second direct current power supply 22 to avoid the currents output from the first direct current power supply 21 and the second direct current power supply 22 passing through the coil.

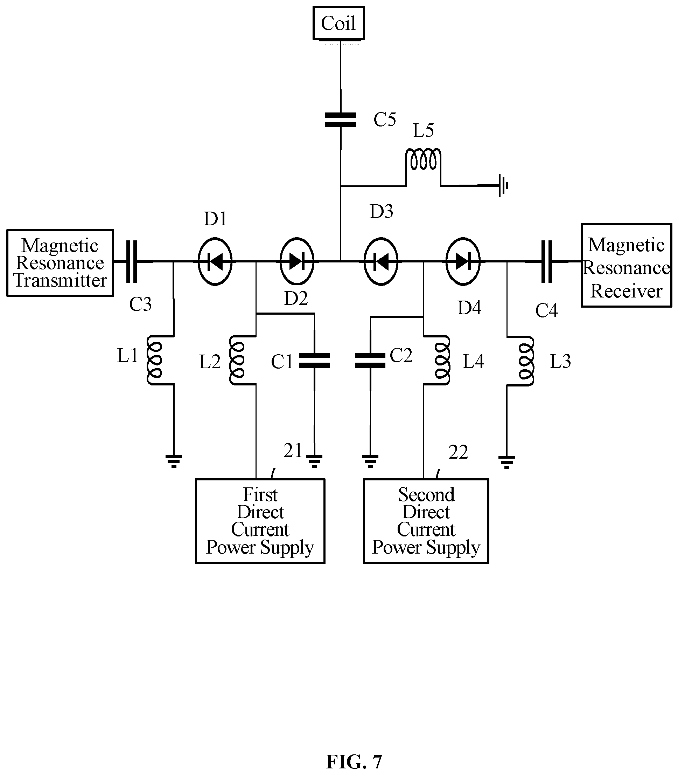

FIG. 7 is a schematic diagram illustrating a structure of a magnetic resonance circuit according to some embodiments of the present disclosure.

As shown in FIG. 7, in some embodiments, when the magnetic resonance transmitter needs to transmit a radio frequency signal to the coil, the first direct current power supply 21 outputs the first turn-on signal. The first diode D1 is conducted along a direction of the second inductor L2, the first diode D1, and the first inductor L1. The second diode D2 is conducted along a direction of the second inductor L2, the second diode D2, and the fifth inductor L5. At this time, the transmission channel from the magnetic resonance transmitter to the coil is opened. At the same time, the second direct current power supply 22 outputs the second cut-off signal, causing the third diode D3 and the fourth diode D4 to be in a cut-off state, i.e., the coil to the magnetic resonance receiver is in the cut-off state. The magnetic resonance transmitter transmits the radio frequency signal to the coil along a direction of the third capacitor C3, the first diode D1, the second diode D2, and the fifth capacitor C5. Since the first inductor L1, the second inductor L2, and the fifth inductor L5 have a function of isolating an alternating current signal, the radio frequency signal may be avoided from escaping from the transmission path during transmission, and a link loss of the radio frequency signal is reduced. Moreover, since the third capacitor C3 can isolate a direct current, the direct current output from the first direct current power supply 21 cannot reach the magnetic resonance transmitter.

When the coil is required to send the collected magnetic resonance signal to the magnetic resonance receiver, the second direct current power supply 22 outputs the second turn-on signal. The third diode D3 is conducted along a direction of the fourth inductor L4, the third diode D3, and the fifth inductor L5. The fourth diode D4 is conducted along a direction of the fourth inductor L4, the fourth diode D4, and the third inductor L3. At this time, the transmission channel from the coil to the magnetic resonance receiver is opened. At the same time, the first direct current power supply 21 outputs the first cut-off signal, causing the first diode D1 and the second diode D2 to be in the cut-off state, i.e., the magnetic resonance transmitter to the coil is in the cut-off state. The magnetic resonance signal collected by the coil is transmitted to the magnetic resonance receiver along a direction of the fifth capacitor C5, the third diode D3, the fourth diode D4, and the fourth capacitor C4. Since the third inductor L3, the fourth inductor L4, and the fifth inductor L5 have the function of isolating an alternating current signal, the collected signal may be avoided from escaping from the transmission path during transmission, and the link loss of the collected signal is reduced. Moreover, since the fourth capacitor C4 can isolate a direct current, the direct current output from the second direct current power supply 22 cannot reach the magnetic resonance receiver.

By providing the third capacitor C3, the fourth capacitor C4, and the fifth capacitor C5 in the magnetic resonance circuit, the direct currents can be effectively avoided from passing through the magnetic resonance transmitter, the magnetic resonance receiver, and the coil, thereby protecting devices in the magnetic resonance system and improving the safety of the magnetic resonance system.

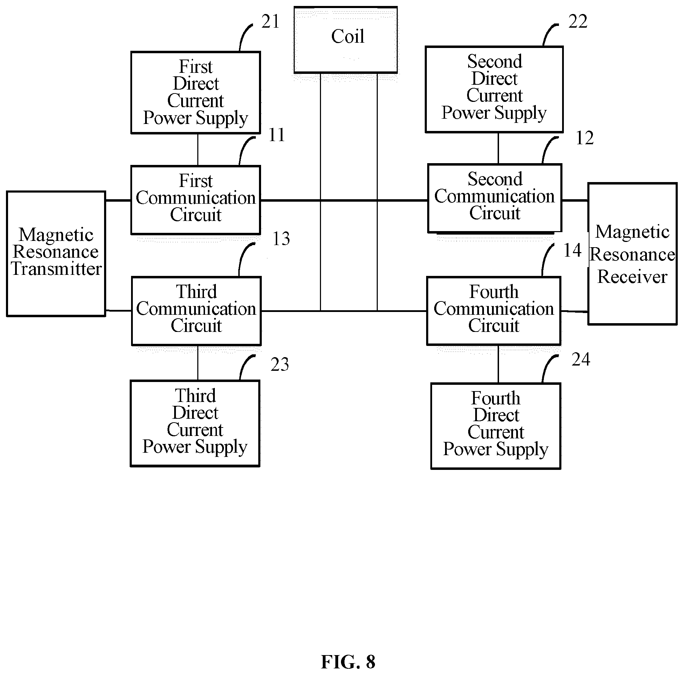

FIG. 8 is a schematic diagram illustrating a structure of a magnetic resonance circuit according to some embodiments of the present disclosure.

As shown in FIG. 8, in some embodiments, the communication circuit 10 may further include a third communication circuit 13 and a fourth communication circuit 14. The control circuit 20 may include a first control circuit and a second control circuit. The first communication circuit 11 and the third communication circuit 13 may be used to transmit the radio frequency signal transmitted by the magnetic resonance transmitter to the coil. The second communication circuit 12 and the fourth communication circuit 14 may be used to transmit the magnetic resonance signal collected by the coil to the magnetic resonance receiver. The first control circuit may be used to send a first control signal to the communication circuit 10 to control the first communication circuit 11 or the second communication circuit 12 to turn on or cut off. The second control circuit may be used to send a second control signal to the communication circuit 10 to control the third communication circuit 13 or the fourth communication circuit 14 to turn on or cut off.

In some embodiments, there may be two transmission channels included between the magnetic resonance transmitter and the coil, i.e., the first communication circuit 11 and the third communication circuit 13. There may also be two transmission channels between the coil and the magnetic resonance receiver, i.e., the second communication circuit 12 and the fourth communication circuit 14. In some embodiments, the first control circuit may be used to send the first control signal to the communication circuit 10 to control the first communication circuit 11 to be turned on or the second communication circuit 12 to be turned on or cut off. The second control circuit may be used to send the second control signal to the communication circuit 10 to control the third communication circuit 13 or the fourth communication circuit 14 to be turned on or cut off.

In some embodiments, the first control circuit may include the first direct current power supply 21 and the second direct current power supply 22. The second control circuit includes a third direct current power supply 23 and a fourth direct current power supply 24. Specifically, as shown in FIG. 8, the first communication circuit 11 and the third communication circuit 13 are respectively connected to the magnetic resonance transmitter and the coil. The second communication circuit 12 and the fourth communication circuit 14 are respectively connected to the coil and the magnetic resonance receiver. The first direct current power supply 21 is connected to the first communication circuit 11, the second direct current power supply 22 is connected to the second communication circuit 12, the third direct current power supply 23 is connected to the third communication circuit 13, and the fourth direct current power supply 24 is connected to the fourth communication circuit 14. The first direct current power supply 21, the second direct current power supply 22, the third direct current power supply 23, and the fourth direct current power supply 24 are respectively used to control the turn-on and cut-off of the first communication circuit 11, the second communication circuit 12, the third communication circuit 13, and the fourth communication circuit 14.

It should be noted that the structures and functions of the third communication circuit 13 and the fourth communication circuit 14 are similar to structures and functions of the first communication circuit 11 and the second communication circuit 12, and structures of the third direct current power supply 23 and the fourth direct current power supply 24 are similar to the structures and functions of the first direct current power supply 21 and the second direct current power supply 22, which may be found in the relevant descriptions of FIG. 2-FIG. 7 of the present disclosure, and will not be repeated herein.

In some embodiments, when the first direct current power supply 21 and the third direct current power supply 23 output the first turn-on signal and a third turn-on signal, the first communication circuit 11 and the third communication circuit 13 are conducted. When the second direct current power supply 22 and the fourth direct current power supply 24 output the second cutoff signal and the fourth cut-off signal, the second communication circuit 12 and the fourth communication circuit 14 are cut off. At this time, the magnetic resonance transmitter and the coil are in a dual-channel communication state, and the magnetic resonance transmitter transmits two radio frequency signals to the coil. In some embodiments, when the second direct current power supply 22 and the fourth direct current power supply 24 output the second turn-on signal and the fourth turn-on signal, the second communication circuit 12 and the fourth communication circuit 14 are conducted; and when the first direct current power supply 21 and the third direct current power supply 23 output the first cut-off signal and a third cut-off signal, the first communication circuit 11 and the third communication circuit 13 are cut off. At this time, the coil and the magnetic resonance transmitter are in the dual-channel communication state, and the coil collects the two magnetic resonance signals and transmits them to the magnetic resonance receiver.

In some embodiments, through the two transmission channels between the magnetic resonance transmitter and the coil and the two transmission channels between the coil and the magnetic resonance receiver, the magnetic resonance transmitter may respectively output two radio frequency signals with a phase difference (e.g., 90 degrees) to the magnetic resonance radio frequency power transceiver. In such cases, the magnetic resonance system includes a dual-channel transmission channel. In the magnetic resonance system, the magnetic resonance transmitter may adjust the phase and amplitude of the radio frequency signal through various types of related devices (e.g., a power divider, a phase shifter, an attenuator, etc.), thereby generating a multi-path parallel radio frequency signal with adjustable frequency, phase, and amplitude. In an ultra-high field magnetic resonance system, a multi-path radio frequency signal can solve problems such as emission field inhomogeneity and radio frequency energy absorption, and can improve a signal-to-noise ratio of a magnetic resonance image while speeding up the velocity of imaging. The dual-channel transmission channel of the embodiments of the present disclosure may respectively output two radio frequency signals with a phase difference to the magnetic resonance radio frequency power transceiver, thereby effectively improving the operating efficiency of transmission and reception of the radio frequency.

In some embodiments, the first communication circuit 11 may be used to transmit a first radio frequency signal generated by the magnetic resonance transmitter to the coil, the second communication circuit 12 may be used to transmit the first magnetic resonance signal emitted by the coil to the magnetic resonance receiver, the third communication circuit 13 may be used to transmit a second radio frequency signal generated by the magnetic resonance transmitter to the coil, and the fourth communication circuit 14 may be used to transmit a second magnetic resonance signal emitted by the coil to the magnetic resonance receiver. The phase difference between the first radio frequency signal and the second radio frequency signal is 90 degrees.

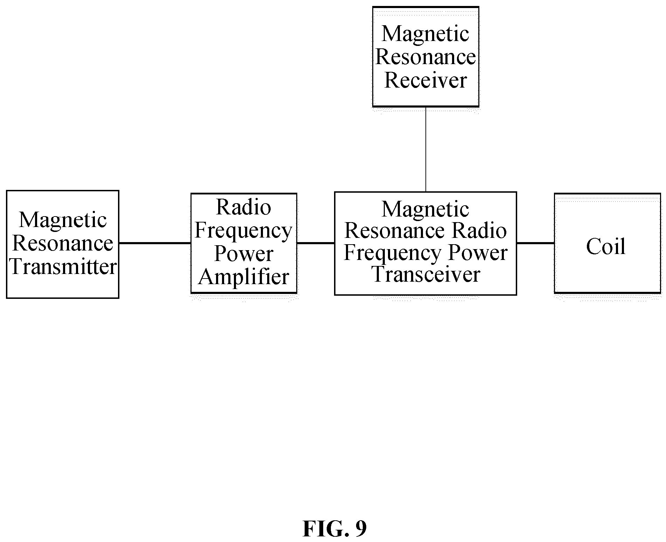

FIG. 9 is a schematic diagram illustrating a structure of a magnetic resonance system according to some embodiments of the present disclosure.

As shown in FIG. 9, in some embodiments, the magnetic resonance system may further include a radio frequency power amplifier for receiving the radio frequency signal output from the magnetic resonance transmitter and performing a power amplification. One end of the radio frequency power amplifier is connected to the magnetic resonance transmitter and another end is connected to the magnetic resonance radio frequency power transceiver.

In some embodiments, if the magnetic resonance radio frequency power transceiver is in the transmitting state, i.e., in the first operating mode, the magnetic resonance transmitter outputs the radio frequency signal to the radio frequency power amplifier, and the radio frequency power amplifier receives the radio frequency signal and performs the power amplification on the radio frequency signal, thereby avoiding a situation in which the detection results are not sufficiently accurate due to the power of the radio frequency signal being too low. If the power of the radio frequency signal is too low, the radio frequency signal may not be able to resonate with the nuclide and cannot generate the magnetic resonance signal. In some embodiments, in order to be suitable for the magnetic resonance environment in which the magnetic resonance system is applied and to support the transmission and power amplification of a plurality of radio frequency signals corresponding to different kinds of nuclides, the radio frequency power amplifier needs to have a large broadband power and bandwidth. Merely by way of example, the broadband power of the radio frequency power amplifier may be 1000 W and a bandwidth of the radio frequency power amplifier may be 30-405 MHz.

In some embodiments, the radio frequency power amplifier may also filter the radio frequency signal output from the magnetic resonance transmitter. The magnetic resonance transmitter may generate a plurality of radio frequency signals corresponding to different kinds of nuclides. If one of the nuclides (e.g., hydrogen nucleus) needs to be detected, the radio frequency power amplifier may amplify the radio frequency signal corresponding to the nuclide and transmit the radio frequency signal to the coil via the magnetic resonance radio frequency power transceiver. In some embodiments, the radio frequency power amplifier may be a single frequency point power amplifier, thereby amplifying the radio frequency signal at a particular frequency point and filtering out the radio frequency signals at other frequency points. In some embodiments, the radio frequency power amplifier may include a plurality of single frequency point power amplifiers, thereby performing the transmission and power amplification of a plurality of radio frequency signals corresponding to different kinds of nuclides with different frequencies.

In some embodiments, the radio frequency power amplifier may also not filter the radio frequency signal output from the magnetic resonance transmitter.

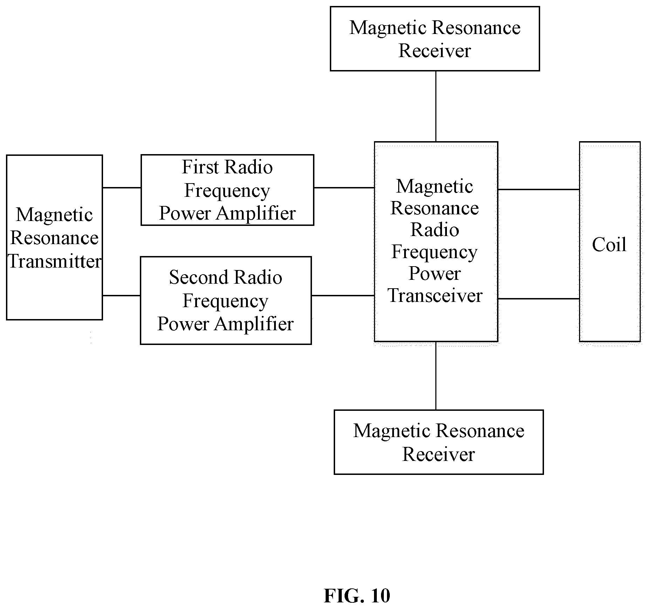

FIG. 10 is a schematic diagram illustrating a structure of a magnetic resonance system according to some embodiments of the present disclosure.

In some embodiments, if the magnetic resonance system includes a dual-channel transmission channel, the magnetic resonance transmitter may be used to output two radio frequency signals with a phase difference of 90 degrees to the magnetic resonance radio frequency power transceiver. Exemplarily, in the magnetic resonance system, there are dual channels included between the magnetic resonance transmitter and the coil, and between the coil and the magnetic resonance receiver. The magnetic resonance transmitter outputs two transmission signals with a phase difference of 90 degrees and transmits them to the magnetic resonance radio frequency power transceiver through the dual-channel between the magnetic resonance transmitter and the coil, and finally transmits them to the coil.

As shown in FIG. 10, the magnetic resonance system may include a first radio frequency power amplifier and a second radio frequency power amplifier. The first radio frequency power amplifier and the second radio frequency power amplifier are connected in parallel between the magnetic resonance transmitter and the magnetic resonance radio frequency power transceiver, i.e., one end of the first radio frequency power amplifier and one end of the second radio frequency power amplifier are jointly connected to the magnetic resonance transmitter, and another ends of them are jointly connected to the magnetic resonance radio frequency power transceiver.

**In** the first operating mode, the magnetic resonance transmitter transmits two radio frequency signals with a phase difference of 90 degrees to the first radio frequency power amplifier and the second radio frequency power amplifier, respectively. Two high-power radio frequency signals with the phase difference of 90 degrees are generated after power amplification, and they can be sent to the coil through the magnetic resonance radio frequency power transceiver. The coil further generates a magnetic field (e.g., a circularly polarized magnetic field generated through orthogonal excitation) to excite the nuclide to resonate and thereby generating the magnetic resonance signal. In the second operating mode, the coil collects the magnetic resonance signal and sends the magnetic resonance signal via the magnetic resonance radio frequency power transceiver to the magnetic resonance receiver for subsequent processing.

**In** some embodiments, the magnetic resonance radio frequency power transceiver may further include two radio frequency power switches to replace the communication circuit 10 and the control circuit 20. The two radio frequency power switches support the signal transmission of the plurality of radio frequency signals and may be used to determine the operating mode of the magnetic resonance radio frequency power transceiver.

Merely by way of example, the magnetic resonance radio frequency power transceiver may include two radio frequency power switches. Each of the two radio frequency power switches respectively controls one of the dual channels, thereby realizing the switching of the operating modes. Moreover, each of the two radio frequency power switches is capable of implementing a wide bandwidth transmission, i.e., supporting signal transmission of a plurality of frequencies. In some embodiments, each of the two radio frequency power switches may be an independent electronic component supporting a wide bandwidth range, or may be a combination of a plurality of electronic components corresponding to the plurality of frequencies. In some embodiments, to support the signal transmission of the plurality of radio frequency signals corresponding to different kinds of nuclides, i.e., to realize a wide bandwidth transmission, a frequency point supported by each of the two radio frequency power switches is within a range of 100-500 MHz, and a rated power of the radio frequency power switch may be 1,000 W. Merely by way of example, the frequency points supported by each of the two radio frequency power switches may include 105.92 MHz, 110.81 MHz, 376.84 MHz, or 400.55 MHz.

Specifically, the radio frequency power switches in the embodiments of the present disclosure enable the transmission and reception of the different kinds of nuclides (e.g., hydrogen (1H), the carbon (13C), the nitrogen (15N), the fluorine (19F), the sodium (23Na), the phosphorus (31P), and the xenon (129Xe)) in a high-field (e.g., magnetic field strength of 5T and above) magnetic resonance broadband without the need for switching, as well as without the need for switching the radio frequency power switches to match different frequencies.

The magnetic resonance radio frequency power transceiver in the embodiments of the present disclosure may include two radio frequency power switches. The radio frequency power switches support the transmission of signals with a plurality of frequencies, which can be used to determine the operating mode of the magnetic resonance radio frequency power transceiver. The broadband transmission is realized through the radio frequency power switches supporting multiple frequencies without the need to switch the radio frequency power switches to match different frequencies, which reduces the count of switches in the magnetic resonance system, thereby reducing the volume and the hardware cost of the magnetic resonance system.

The beneficial effects provided by the embodiments of the present disclosure include but are not limited to: (1) by the magnetic resonance radio frequency power transceiver and entering the first operating mode or the second operating mode according to the control signal, thereby realizing the switching between the transmission channel between the magnetic resonance transmitter and the coil and the transmission channel between the coil and the magnetic resonance receiver, there is no need to provide different transmission channels for excitations (or reception) of different nuclides, avoiding the use of too many hardware switches; (2) by adopting a magnetic resonance radio frequency power transceiver that supports broadband communication at a plurality of frequency points, the count of transmission channels between the magnetic resonance transmitter and the coil, and between the coil and the magnetic resonance receiver can be effectively reduced, thereby reducing the volume and the hardware cost of the magnetic resonance system and improving the operating efficiency of the transmission and reception of the radio frequency; (3) by respectively controlling the turn-on and cut-off of the first communication circuit and the second communication circuit using the first direct current power supply and the second direct current power supply, there is no need for a complex circuit control structure, the implementation process is simple, and the hardware cost of the control circuit is reduced; (4) by providing inductor elements to isolate radio frequency signals and collect signals during transmission, the losses of the radio frequency signals and the collected magnetic resonance signals during transmission are avoided to ensure the signal quality, thereby enhancing the detection accuracy of the magnetic resonance system; (5) the interferences from signals other than the control signals, the plurality of radio frequency signals, and the plurality of magnetic resonance signals are filtered out using the filter circuit composed of capacitive elements and inductor elements to filter out; (6) by providing the capacitive element in the magnetic resonance circuit, the direct currents are effectively prevented from passing through the magnetic resonance transmitter, the magnetic resonance receiver, and the coil, thereby protecting the devices in the magnetic resonance system and improving the safety of the magnetic resonance system; (7) the dual-channel transmission channel can respectively output two radio frequency signals with a phase difference to the magnetic resonance radio frequency power transceiver, which improves the operating efficiency of the transmission and reception of the radio frequency.

It should be noted that different embodiments may produce different beneficial effects, and in different embodiments, the potential beneficial effects may be any one or a combination of one or more of the above, or any other beneficial effect that may be obtained.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure, and are within the scope of the claims.

Moreover, certain terminology has been used to describe embodiments of the present disclosure. For example, the terms "one embodiment," "an embodiment," and/or "some embodiments" mean that a particular feature, structure, or characteristic described in connection with the embodiment is included in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures, or characteristics may be combined as suitable in one or more embodiments of the present disclosure, provided that the resulting subject-matter falls within the scope of the invention as defined by the claims.

Furthermore, the recited order of processing elements or sequences, or the use of numbers, letters, or other designations thereof, are not intended to limit the claimed processes and methods to any order except as may be specified in the claims. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications that are within the scope of the invention as defined by the claims. For example, although the implementation of various components described above may be embodied in a hardware device, it may also be implemented as a software-only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. This method of disclosure, however, is not to be interpreted as reflecting an intention that the claimed subject matter requires more features than are expressly recited in each claim. Rather, claimed subject matter may lie in less than all features of a single foregoing disclosed embodiment.

In some embodiments, the numbers expressing quantities or properties used to describe and claim certain embodiments of the application are to be understood as being modified in some instances by the term "about," "approximate," or "substantially." For example, "about," "approximate," or "substantially" may indicate ±20% variation of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the count of reported significant digits and by applying ordinary rounding techniques. Notwithstanding that the numerical ranges and parameters setting forth the broad scope of some embodiments of the application are approximations, the numerical values set forth in the specific examples are reported as precisely as practicable.

In closing, it is to be understood that the embodiments of the application disclosed herein are illustrative of the principles of the embodiments of the application. Other modifications that may be employed may be within the scope of the application. Therefore, by way of example, but not of limitation, alternative configurations of the embodiments of the application may be utilized in accordance with the teachings herein. Accordingly, embodiments of the present application are not limited to that precisely as shown and described. In any case, the scope of the invention is defined by the claims.

## Claims

1. A magnetic resonance system, comprising:
a magnetic resonance transmitter configured to generate one or more radio frequency signals;
a magnetic resonance receiver configured to receive one or more magnetic resonance signals collected by one or more coils; and
a magnetic resonance radio frequency power transceiver, including a communication circuit (10) and a control circuit (20), wherein
the communication circuit (10) includes a first communication circuit (11) and a second communication circuit (12), the first communication circuit (11) is configured to transmit the one or more radio frequency signals to the one or more coils, and the second communication circuit (12) is configured to transmit the one or more magnetic resonance signals to the magnetic resonance receiver; and
the control circuit (20) is configured to send one or more control signals to the communication circuit (10) to control the first communication circuit (11) or the second communication circuit (12) to turn on or cut off, wherein the control circuit (20) includes a power supply, the power supply is configured to output a turn-on signal and/or a cut-off signal to control the first communication circuit (11) and the second communication circuit (12) to turn on and/or cut off, the power supply includes a first direct current power supply (21) and a second direct current power supply (22), the first direct current power supply (21) is connected to the first communication circuit (11), the second direct current power supply (22) is connected to the second communication circuit (12), wherein
the first communication circuit (11) includes a first diode (D1) and a second diode (D2), a negative electrode of the first diode (D1) is connected to the magnetic resonance transmitter, a positive electrode of the second diode (D2) is connected to a positive electrode of the first diode (D1), and a negative electrode of the second diode (D2) is connected to the one or more coils;
the second communication circuit (12) includes a third diode (D3) and a fourth diode (D4), a negative electrode of the third diode (D3) is connected to the one or more coils, and a positive electrode of the fourth diode (D4) is connected to a positive electrode of the third diode (D3) and a negative electrode of the fourth diode (D4) is connected to the magnetic resonance receiver;
the first direct current power supply (21) is connected to the positive electrode of the first diode (D1) and the positive electrode of the second diode (D2), the second direct current power supply (22) is connected to the positive electrode of the third diode (D3) and the positive electrode of the fourth diode (D4).

2. The system of claim 1, wherein the one or more radio frequency signals are a plurality of radio frequency signals corresponding to different kinds of nuclides, the one or more magnetic resonance signals are a plurality of magnetic resonance signals sent by the different kinds of nuclides, and the second communication circuit (12) is configured to transmit the plurality of magnetic resonance signals to the magnetic resonance receiver, respectively.

3. The system of claim 2, wherein the magnetic resonance system includes the one or more coils, the one or more coils are provided inside or connected to the magnetic resonance system, and the one or more coils are configured to generate a magnetic field that excites resonance of the different kinds of nuclides and/or to collect the plurality of magnetic resonance signals sent by the different kinds of nuclides.

4. The system of claim 1, wherein the system further comprises a first inductor (L1), a second inductor (L2), a third inductor (L3), a fourth inductor (L4), a fifth inductor (L5), and a capacitive element, wherein
the first inductor (L1) is connected between the magnetic resonance transmitter and a ground end,
the second inductor (L2) is connected between the first direct current power supply (21) and the first communication circuit (11),
the third inductor (L3) is connected between the magnetic resonance receiver and the ground end,
the fourth inductor (L4) is connected between the second direct current power supply (22) and the second communication circuit (12),
the fifth inductor (L5) is connected between the one or more coils and the ground end, and
the capacitive element forms a filter circuit with the second inductor (L2) and the fourth inductor (L4), the filter circuit is configured to filter out interferences from other signals other than the one or more control signals, the plurality of radio frequency signals, and the plurality of magnetic resonance signals.

5. The system of claim 4, wherein the capacitive element includes a first capacitor (C1) and a second capacitor (C2), the first capacitor (C1) is connected to the second inductor (L2), and the second capacitor (C2) is connected to the fourth inductor (L4), wherein the first capacitor (C1) and the second inductor (C2) form a first filter circuit, and the second capacitor (C2) and the fourth inductor (L4) form a second filter circuit.

6. The system of claim 1, further comprising a third capacitor (C3), a fourth capacitor (C4), and a fifth capacitor (C5), wherein
the third capacitor (C3) is connected between the magnetic resonance transmitter and the first communication circuit (11), and the third capacitor (C3) is configured to isolate a first direct current output from the first direct current power supply (21) to avoid the first direct current passing through the magnetic resonance transmitter when the first communication circuit (11) is turned on;
the fourth capacitor (C4) is connected between the magnetic resonance receiver and the second communication circuit (12), and the fourth capacitor (C4) is configured to isolate a second direct current output from the second direct current power supply (22) to avoid the second direct current passing through the magnetic resonance receiver when the second communication circuit (12) is turned on; and
the fifth capacitor (C5) is connected between the one or more coils and the communication circuit (10), and the fifth capacitor (C4) is configured to isolate the first direct current and the second direct current to avoid the first direct current and the second direct current passing through the one or more coils.

7. The system of claim 3, further comprising a radio frequency power amplifier, wherein
the radio frequency power amplifier is configured to receive the plurality of radio frequency signals and perform a power amplification, and
a bandwidth of the radio frequency power amplifier is within a range of 30 to 405 MHz.

8. The system of claim 2, wherein the communication circuit (10) further includes a third communication circuit (13) and a fourth communication circuit (14), and the magnetic resonance transmitter is further configured to output two radio frequency signals with a phase difference of 90 degrees to the magnetic resonance radio frequency power transceiver.

9. The system of claim 8, wherein.
the first communication circuit (11) is configured to transmit a first radio frequency signal generated by the magnetic resonance transmitter to the one or more coils,
the second communication circuit (12) is configured to transmit a first magnetic resonance signal sent from the one or more coils to the magnetic resonance receiver,
the third communication circuit (13) is configured to transmit a second radio frequency signal generated by the magnetic resonance transmitter to the one or more coils, and
the fourth communication circuit (14) is configured to transmit a second magnetic resonance signal sent from the one or more coils to the magnetic resonance receiver,
wherein a phase difference between the first radio frequency signal and the second radio frequency signal is 90 degrees.

10. The system of claim 8, wherein the communication circuit (10) includes two radio frequency power switches, the two radio frequency power switches support signal transmission at multiple frequencies and are configured to control an operating mode of the magnetic resonance radio frequency power transceiver, and a frequency point supported by each of the two radio frequency power switches is within a range of 100-500 MHz.

11. The system of claim 3, wherein the one or more coils includes multiple groups of coils, each group of coils respectively corresponds to the one or more radio frequency signals or the one or more magnetic resonance signals of the different kinds of nuclides.

12. The system of claim 4, wherein one end of the second inductor (L2) is connected to the first direct current power supply (21), and another end of the second inductor (L2) is respectively connected to the positive electrode of the first diode (D1) and the positive electrode of the second diode (D2).

## Patentansprüche

1. Magnetresonanzsystem, umfassend:
einen Magnetresonanzsender, der so konfiguriert ist, dass er ein oder mehrere Hochfrequenzsignale erzeugt;
einen Magnetresonanzempfänger, der so konfiguriert ist, dass er eines oder mehrere von einer oder mehreren Spulen erfasste Magnetresonanzsignale empfängt; und
einen Magnetresonanz-Hochfrequenz-Leistungstransceiver, der eine Kommunikationsschaltung (10) und eine Steuerschaltung (20) beinhaltet, wobei die Kommunikationsschaltung (10) eine erste Kommunikationsschaltung (11) und eine zweite Kommunikationsschaltung (12) beinhaltet, die erste Kommunikationsschaltung (11) zur Übertragung des einen oder der mehreren Hochfrequenzsignale an die eine oder mehrere Spulen konfiguriert ist und die zweite Kommunikationsschaltung (12) zur Übertragung des einen oder der mehreren Magnetresonanzsignale an den Magnetresonanzempfänger konfiguriert ist; und
die Steuerschaltung (20) so konfiguriert ist, dass sie ein oder mehrere Steuersignale an die Kommunikationsschaltung (10) sendet, um die erste Kommunikationsschaltung (11) oder die zweite Kommunikationsschaltung (12) zu steuern, um ein- oder auszuschalten, wobei die Steuerschaltung (20) eine Stromversorgung beinhaltet, wobei die Stromversorgung so konfiguriert ist, dass sie ein Einschaltsignal und/oder ein Abschaltsignal zur Steuerung der ersten Kommunikationsschaltung (11) und der zweiten Kommunikationsschaltung (12) zum Einschalten und/oder Ausschalten ausgibt, die Stromversorgung eine erste Gleichstromversorgung (21) und eine zweite Gleichstromversorgung (22) beinhaltet, wobei die erste Gleichstromversorgung (21) mit der ersten Kommunikationsschaltung (11) verbunden ist, die zweite Gleichstromversorgung (22) mit der zweiten Kommunikationsschaltung (12) verbunden ist, wobei
die erste Kommunikationsschaltung (11) eine erste Diode (D1) und eine zweite Diode (D2) beinhaltet, wobei eine negative Elektrode der ersten Diode (D1) mit dem Magnetresonanzsender verbunden ist, eine positive Elektrode der zweiten Diode (D2) mit einer positiven Elektrode der ersten Diode (D1) verbunden ist und eine negative Elektrode der zweiten Diode (D2) mit einer oder mehreren Spulen verbunden ist;
die zweite Kommunikationsschaltung (12) eine dritte Diode (D3) und eine vierte Diode (D4) beinhaltet, wobei eine negative Elektrode der dritten Diode (D3) mit der einen oder den mehreren Spulen verbunden ist und eine positive Elektrode der vierten Diode (D4) mit einer positiven Elektrode der dritten Diode (D3) verbunden ist und eine negative Elektrode der vierten Diode (D4) mit dem Magnetresonanzempfänger verbunden ist;
die erste Gleichstromversorgung (21) mit der positiven Elektrode der ersten Diode (D1) und der positiven Elektrode der zweiten Diode (D2) verbunden ist; die zweite Gleichstromversorgung (22) mit der positiven Elektrode der dritten Diode (D3) und der positiven Elektrode der vierten Diode (D4) verbunden ist.

2. System nach Anspruch 1, wobei es sich bei dem einen oder mehreren Hochfrequenzsignalen um eine Vielzahl von Hochfrequenzsignalen handelt, die verschiedenen Nuklidenarten entsprechen, bei dem einen oder mehreren Magnetresonanzsignalen um eine Vielzahl von Magnetresonanzsignalen handelt, die von den verschiedenen Nuklidenarten ausgesendet werden, und die zweite Kommunikationsschaltung (12) so konfiguriert ist, dass sie die Vielzahl von Magnetresonanzsignalen jeweils an den Magnetresonanzempfänger überträgt.

3. System nach Anspruch 2, wobei das Magnetresonanzsystem die eine oder mehreren Spulen beinhaltet, die eine oder mehreren Spulen innerhalb des Magnetresonanzsystems angeordnet oder mit diesem verbunden sind und die eine oder mehreren Spulen so konfiguriert sind, dass sie ein Magnetfeld erzeugen, das Resonanz der verschiedenen Nuklidenarten anregt, und/oder die Vielzahl der von den verschiedenen Nukliden gesendeten magnetischen Resonanzsignale sammeln.

4. System nach Anspruch 1, wobei das System ferner einen ersten Induktor (L1), einen zweiten Induktor (L2), einen dritten Induktor (L3), einen vierten Induktor (L4), einen fünften Induktor (L5) und ein kapazitives Element umfasst, wobei
der erste Induktor (L1) zwischen dem Magnetresonanzsender und einem Masseanschluss angeschlossen ist,
der zweite Induktor (L2) zwischen der ersten Gleichstromversorgung (21) und der ersten Kommunikationsschaltung (11) angeschlossen ist,
der dritte Induktor (L3) zwischen dem Magnetresonanzempfänger und dem Masseanschluss angeschlossen ist,
der vierte Induktor (L4) zwischen der zweiten Gleichstromversorgung (22) und der zweiten Kommunikationsschaltung (12) angeschlossen ist,
der fünfte Induktor (L5) zwischen der einen oder den mehreren Spulen und dem Masseanschluss angeschlossen ist und
das kapazitive Element mit dem zweiten Induktor (L2) und dem vierten Induktor (L4) einen Filterkreis bildet, wobei der Filterkreis so konfiguriert ist, dass er Störungen durch andere Signale als das eine oder die mehreren Steuersignale, die Vielzahl von Hochfrequenzsignalen und die Vielzahl von Magnetresonanzsignalen herausfiltert.

5. System nach Anspruch 4, wobei das kapazitive Element einen ersten Kondensator (C1) und einen zweiten Kondensator (C2) beinhaltet, wobei der erste Kondensator (C1) mit dem zweiten Induktor (L2) verbunden ist und der zweite Kondensator (C2) mit dem vierten Induktor (L4) verbunden ist, wobei der erste Kondensator (C1) und der zweite Induktor (C2) einen ersten Filterkreis bilden und der zweite Kondensator (C2) und der vierte Induktor (L4) einen zweiten Filterkreis bilden.

6. System nach Anspruch 1, ferner umfassend einen dritten Kondensator (C3), einen vierten Kondensator (C4) und einen fünften Kondensator (C5), wobei
der dritte Kondensator (C3) zwischen dem Magnetresonanzsender und der ersten Kommunikationsschaltung (11) angeschlossen ist und der dritte Kondensator (C3) so konfiguriert ist, dass er einen ersten Gleichstromausgang von der ersten Gleichstromversorgung (21) isoliert, um zu vermeiden, dass der erste Gleichstrom durch den Magnetresonanzsender fließt, wenn die erste Kommunikationsschaltung (11) eingeschaltet wird;
der vierte Kondensator (C4) zwischen dem Magnetresonanzempfänger und der zweiten Kommunikationsschaltung (12) angeschlossen ist und der vierte Kondensator (C4) so konfiguriert ist, dass er einen zweiten Gleichstromausgang von der zweiten Gleichstromversorgung (22) isoliert, um zu verhindern, dass der zweite Gleichstrom durch den Magnetresonanzempfänger fließt, wenn die zweite Kommunikationsschaltung (12) eingeschaltet ist; und
der fünfte Kondensator (C5) zwischen der einen oder den mehreren Spulen und der Kommunikationsschaltung (10) angeschlossen ist und der fünfte Kondensator (C4) so konfiguriert ist, dass er den ersten Gleichstrom und den zweiten Gleichstrom isoliert, um zu vermeiden, dass der erste Gleichstrom und der zweite Gleichstrom durch die eine oder mehrere Spulen fließen.

7. System nach Anspruch 3, ferner umfassend einen Hochfrequenz-Leistungsverstärker, wobei
der Hochfrequenz-Leistungsverstärker so konfiguriert ist, dass er die Vielzahl von Hochfrequenzsignalen empfängt und eine Leistungsverstärkung durchführt, und
eine Bandbreite des Hochfrequenz-Leistungsverstärkers im Bereich von 30 bis 405 MHz liegt.

8. System nach Anspruch 2, wobei die Kommunikationsschaltung (10) ferner eine dritte Kommunikationsschaltung (13) und eine vierte Kommunikationsschaltung (14) beinhaltet und der Magnetresonanzsender ferner so konfiguriert ist, dass er zwei Hochfrequenzsignale mit einer Phasendifferenz von 90 Grad an den Magnetresonanz-Hochfrequenz-Leistungstransceiver ausgibt.

9. System nach Anspruch 8, wobei:
die erste Kommunikationsschaltung (11) so konfiguriert ist, dass sie ein erstes vom Magnetresonanzsender erzeugtes Hochfrequenzsignal an die eine oder mehrere Spulen überträgt,
die zweite Kommunikationsschaltung (12) so konfiguriert ist, dass sie ein erstes von der einen oder den mehreren Spulen gesendete Magnetresonanzsignal an den Magnetresonanzempfänger überträgt,
die dritte Kommunikationsschaltung (13) so konfiguriert ist, dass sie ein zweites, vom Magnetresonanzsender erzeugtes Hochfrequenzsignal an die eine oder mehrere Spulen überträgt, und
die vierte Kommunikationsschaltung (14) so konfiguriert ist, dass sie ein zweites von der einen oder den mehreren Spulen gesendete Magnetresonanzsignal an den Magnetresonanzempfänger überträgt,
wobei eine Phasendifferenz zwischen dem ersten Hochfrequenzsignal und dem zweiten Hochfrequenzsignal 90 Grad beträgt.

10. System nach Anspruch 8, wobei die Kommunikationsschaltung (10) zwei Hochfrequenz-Leistungsschalter beinhaltet, die beiden Hochfrequenz-Leistungsschalter Signalübertragung bei mehreren Frequenzen unterstützen und so konfiguriert sind, dass sie einen Betriebsmodus des Magnetresonanz-Hochfrequenz-Leistungstransceivers steuern, und ein von jedem der beiden Hochfrequenz-Leistungsschalter unterstützter Frequenzpunkt im Bereich von 100-500 MHz liegt.

11. System nach Anspruch 3, wobei die eine oder mehreren Spulen mehrere Spulengruppen beinhalten, wobei jede Spulengruppe jeweils dem einem oder den mehreren Hochfrequenzsignalen oder dem einem oder den mehreren Magnetresonanzsignalen verschiedener Nuklide entspricht.

12. System nach Anspruch 4, wobei ein Ende des zweiten Induktors (L2) mit der ersten Gleichstromversorgung (21) verbunden ist und das andere Ende des zweiten Induktors (L2) jeweils mit der positiven Elektrode der ersten Diode (D1) und der positiven Elektrode der zweiten Diode (D2) verbunden ist.

## Revendications

1. Système à résonance magnétique, comprenant :
un émetteur à résonance magnétique configuré pour générer un ou plusieurs signaux radiofréquence ;
un récepteur à résonance magnétique configuré pour recevoir un ou plusieurs signaux de résonance magnétique recueillis par une ou plusieurs bobines ; et
un émetteur-récepteur de puissance radiofréquence à résonance magnétique, incluant un circuit de communication (10) et un circuit de commande (20), dans lequel le circuit de communication (10) inclut un premier circuit de communication (11) et un deuxième circuit de communication (12), le premier circuit de communication (11) est configuré pour transmettre les un ou plusieurs signaux radiofréquence aux une ou plusieurs bobines, et le deuxième circuit de communication (12) est configuré pour transmettre les un ou plusieurs signaux de résonance magnétique au récepteur à résonance magnétique ; et
le circuit de commande (20) est configuré pour envoyer un ou plusieurs signaux de commande au circuit de communication (10) pour commander l'activation ou la désactivation du premier circuit de communication (11) ou du deuxième circuit de communication (12), dans lequel le circuit de commande (20) inclut une alimentation, l'alimentation est configurée pour fournir un signal d'activation et/ou un signal de désactivation pour commander l'activation et/ou la désactivation du premier circuit de communication (11) et du deuxième circuit de communication (12), l'alimentation inclut une première alimentation en courant continu (21) et une seconde alimentation en courant continu (22), la première alimentation en courant continu (21) est connectée au premier circuit de communication (11), la seconde alimentation en courant continu (22) est connectée au deuxième circuit de communication (12).
le premier circuit de communication (11) inclut une première diode (D1) et une deuxième diode (D2), une électrode négative de la première diode (D1) est connectée à l'émetteur à résonance magnétique, une électrode positive de la deuxième diode (D2) est connectée à une électrode positive de la première diode (D1), et une électrode négative de la deuxième diode (D2) est connectée aux une ou plusieurs bobines ;
le deuxième circuit de communication (12) inclut une troisième diode (D3) et une quatrième diode (D4), une électrode négative de la troisième diode (D3) est connectée aux une ou plusieurs bobines, et une électrode positive de la quatrième diode (D4) est connectée à une électrode positive de la troisième diode (D3) et une électrode négative de la quatrième diode (D4) est connectée au récepteur à résonance magnétique ;
la première alimentation en courant continu (21) est connectée à l'électrode positive de la première diode (D1) et à l'électrode positive de la deuxième diode (D2), la seconde alimentation en courant continu (22) est connectée à l'électrode positive de la troisième diode (D3) et à l'électrode positive de la quatrième diode (D4).

2. Système selon la revendication 1, dans lequel les un ou plusieurs signaux radiofréquence sont une pluralité de signaux radiofréquence correspondant à différents types de nucléides, les un ou plusieurs signaux de résonance magnétique sont une pluralité de signaux de résonance magnétique envoyés par les différents types de nucléides, et le deuxième circuit de communication (12) est configuré pour transmettre la pluralité de signaux de résonance magnétique au récepteur à résonance magnétique, respectivement.

3. Système selon la revendication 2, dans lequel le système à résonance magnétique inclut les une ou plusieurs bobines, les une ou plusieurs bobines sont prévues à l'intérieur du système à résonance magnétique ou connectées à celui-ci, et les une ou plusieurs bobines sont configurées pour générer un champ magnétique qui excite la résonance des différents types de nucléides et/ou pour recueillir la pluralité de signaux de résonance magnétique envoyés par les différents types de nucléides.

4. Système selon la revendication 1, dans lequel le système comprend en outre un premier inducteur (L1), un deuxième inducteur (L2), un troisième inducteur (L3), un quatrième inducteur (L4), un cinquième inducteur (L5) et un élément capacitif, dans lequel
le premier inducteur (L1) est connecté entre l'émetteur à résonance magnétique et une extrémité de masse,
le deuxième inducteur (L2) est connecté entre la première alimentation en courant continu (21) et le premier circuit de communication (11),
le troisième inducteur (L3) est connecté entre le récepteur à résonance magnétique et l'extrémité de masse,
le quatrième inducteur (L4) est connecté entre la seconde alimentation en courant continu (22) et le deuxième circuit de communication (12),
le cinquième inducteur (L5) est connecté entre les une ou plusieurs bobines et l'extrémité de masse, et
l'élément capacitif forme un circuit de filtrage avec la deuxième inducteur (L2) et le quatrième inducteur (L4), le circuit de filtrage est configuré pour filtrer les interférences provenant d'autres signaux que les un ou plusieurs signaux de commande, la pluralité de signaux radiofréquence et la pluralité de signaux de résonance magnétique.

5. Système selon la revendication 4, dans lequel l'élément capacitif inclut un premier condensateur (C1) et un deuxième condensateur (C2), le premier condensateur (C1) est connecté au deuxième inducteur (L2), et le deuxième condensateur (C2) est connecté au quatrième inducteur (L4), dans lequel le premier condensateur (C1) et le deuxième inducteur (C2) forment un premier circuit de filtrage, et le deuxième condensateur (C2) et le quatrième inducteur (L4) forment un deuxième circuit de filtrage.

6. Système selon la revendication 1, comprenant en outre un troisième condensateur (C3), un quatrième condensateur (C4) et un cinquième condensateur (C5), dans lequel
le troisième condensateur (C3) est connecté entre l'émetteur à résonance magnétique et le premier circuit de communication (11), et le troisième condensateur (C3) est configuré pour isoler une première sortie de courant continu provenant de la première alimentation en courant continu (21) pour éviter que le premier courant continu ne passe à travers l'émetteur à résonance magnétique lorsque le premier circuit de communication (11) est activé ;
le quatrième condensateur (C4) est connecté entre le récepteur à résonance magnétique et le deuxième circuit de communication (12), et le quatrième condensateur (C4) est configuré pour isoler une deuxième sortie de courant continu provenant de la seconde alimentation en courant continu (22) pour éviter que le second courant continu ne passe à travers le récepteur à résonance magnétique lorsque le deuxième circuit de communication (12) est activé ; et
le cinquième condensateur (C5) est connecté entre les une ou plusieurs bobines et le circuit de communication (10), et le cinquième condensateur (C4) est configuré pour isoler le premier courant continu et le second courant continu pour éviter que le premier courant continu et le second courant continu ne passent à travers les une ou plusieurs bobines.

7. Système selon la revendication 3, comprenant en outre un amplificateur de puissance radiofréquence, dans lequel
l'amplificateur de puissance radiofréquence est configuré pour recevoir la pluralité de signaux radiofréquence et effectuer une amplification de puissance, et
une bande passante de l'amplificateur de puissance radiofréquence se situe dans une plage de 30 à 405 MHz.

8. Système selon la revendication 2, dans lequel le circuit de communication (10) inclut en outre un troisième circuit de communication (13) et un quatrième circuit de communication (14), et l'émetteur à résonance magnétique est en outre configuré pour produire en sortie deux signaux radiofréquence avec une différence de phase de 90 degrés vers l'émetteur-récepteur de puissance radiofréquence à résonance magnétique.

9. Système selon la revendication 8, dans lequel :
le premier circuit de communication (11) est configuré pour transmettre un premier signal radiofréquence généré par l'émetteur à résonance magnétique aux une ou plusieurs bobines,
le deuxième circuit de communication (12) est configuré pour transmettre un premier signal de résonance magnétique envoyé par les une ou plusieurs bobines au récepteur à résonance magnétique,
le troisième circuit de communication (13) est configuré pour transmettre un deuxième signal radiofréquence généré par l'émetteur à résonance magnétique aux une ou plusieurs bobines, et
le quatrième circuit de communication (14) est configuré pour transmettre un deuxième signal de résonance magnétique envoyé par les une ou plusieurs bobines au récepteur à résonance magnétique,
dans lequel une différence de phase entre le premier signal radiofréquence et le deuxième signal radiofréquence est de 90 degrés.

10. Système selon la revendication 8, dans lequel le circuit de communication (10) inclut deux commutateurs de puissance radiofréquence, les deux commutateurs de puissance radiofréquence prennent en charge la transmission de signaux à de multiples fréquences et sont configurés pour commander un mode de fonctionnement de l'émetteur-récepteur de puissance radiofréquence à résonance magnétique, et un point de fréquence pris en charge par chacun des deux commutateurs de puissance radiofréquence se situe dans une plage de 100 à 500 MHz.

11. Système selon la revendication 3, dans lequel les une ou plusieurs bobines incluent de multiples groupes de bobines, chaque groupe de bobines correspondant respectivement aux un ou plusieurs signaux radiofréquence ou aux un ou plusieurs signaux de résonance magnétique des différents types de nucléides.

12. Système selon la revendication 4, dans lequel une extrémité du deuxième inducteur (L2) est connectée à la première alimentation en courant continu (21), et une autre extrémité du deuxième inducteur (L2) est respectivement connectée à l'électrode positive de la première diode (D1) et à l'électrode positive de la deuxième diode (D2).
